# EUROPÄISCHE PATENTSCHRIFT

(11) **EP 2 044 882 B1**
(45) Veröffentlichungstag und Bekanntmachung des Hinweises auf die Patenterteilung: **29.12.2010**
(21) Anmeldenummer: 08156362.9
(22) Anmeldetag: 16.05.2008
(51) Int. Cl.: A61B 5/0408

(54) **Vorrichtung zur Erfassung und Übertragung von elektrischen Impulsen**
Electrical impulse recording and transmitting device
Dispositif de détection et de transmission d'impulsions électriques

(30) Priorität: 05.10.2007 DE 102007047690
(43) Veröffentlichungstag der Anmeldung: 08.04.2009
(73) Patentinhaber: Dräger Medical GmbH, 23558 Lübeck (DE)
(72) Erfinder: Dr. Grassl, Thomas, 23560 Lübeck (DE)
(74) Vertreter: UEXKÜLL & STOLBERG

(56) Entgegenhaltungen:
- DE-A1- 3 637 956
- US-A- 4 353 372

## Beschreibung

Die vorliegende Erfindung betrifft eine Vorrichtung zur Erfassung und Übertragung von elektrischen Impulsen von der Körperoberfläche eines Patienten an eine die elektrischen Impulse verarbeitende Einrichtung, umfassend wenigstens zwei elektrische Leiter, von denen wenigstens zwei elektrische Leiter eine unterschiedliche Länge aufweisen, wenigstens eine die wenigstens zwei elektrischen Leiter umgebende elektrische Isolierung, einen Verbindungsanschluss zur Verbindung mit der die elektrischen Impulse verarbeitende Einrichtung und eine Ausnehmung in der elektrischen Isolierung der wenigstens zwei elektrischen Leiter, so dass ein elektrischer Kontakt mit der Hautoberfläche des Patienten herstellbar ist.

Vorrichtungen zur Erfassung und Übertragung von elektrischen Impulsen, z. B. EEG-, EKG- und EIT-Kabel, werden beispielsweise für elektrokardiographische Messungen an Patienten eingesetzt, um Informationen über die Herzleistung zu erhalten. Hierzu werden Kontakte auf die Hautoberfläche aufgelegt. Die Summe dieser Einzelpotentiale kann an der Körperoberfläche gemessen werden, wobei es sich um Messsignale mit sehr kleinen Frequenzen, z. B. von 0,1 bis 140 Hz, im nV- und µA-Bereich handelt. Darüber hinaus können mit der gleichen Vorrichtung unter Einspeisung eines elektrischen Wechselstromes mit einer Frequenz von beispielsweise 40 kHz mit der so genannten Impedanzmethode die Atemparameter bestimmt werden.

Bekannte EEG-, EKG- und EIT-Kabel verfügen über eine mehrfache Abschirmung gegen externe Störungen, insbesondere elektromagnetische Störungen, weil von den Störungen induzierte elektrische Spannungen und Ströme (Artefakte) die schwachen Messsignale verfälschen. Derartige EEG-, EKG- und EIT-Kabel sind jedoch in der Herstellung teuer, so dass diese aus wirtschaftlichen Gründen nicht zur einmaligen Verwendung bei Patienten eingesetzt werden können.

Aus der US 4 353 372 ist eine Vorrichtung zur Erfassung und Übertragung von elektrischen Impulsen bekannt. Mehrere elektrische Leiter unterschiedlicher Länge mit einer elektrischen Isolierung sind an einem ersten Ende an einem Verbindungsanschluss zur Verbindung mit der die elektrischen Impulse verarbeitende Einrichtung fixiert. Die zweiten Enden der Leiter mit unterschiedlicher Länge sind mit Kontakten zum Auflegen auf die Hautoberfläche eines Patienten versehen. Aufgrund der unterschiedlichen Längen der Leiter ist zwischen den Elektroden und dem Verbindungsanschluss ein unterschiedlicher elektrischer Widerstand vorhanden. Der elektrische Widerstand der Leiter ist direkt proportional zur Länge der elektrischen Leiter, d. h. das Verhältnis aus dem elektrischen Widerstand und der Länge der Leiter ist konstant. Nachteiligerweise induzieren externe Störungen, insbesondere elektromagnetische Störungen, in den Leitern aufgrund des unterschiedlichen elektrischen Widerstandes der Leiter verschiedene elektrische Spannungen und Ströme (Artefakte), welche von der die elektrischen Impulse verarbeitende Einrichtung, beispielsweise mittels hinterlegter Algorithmen, nicht oder nur teilweise herausgefiltert werden können. Damit ist eine zuverlässige und sichere medizinische Auswertung der Messsignale nicht gewährleistet.

Die Aufgabe der vorliegenden Erfindung besteht deshalb darin, eine Vorrichtungen zur Erfassung und Übertragung von elektrischen Impulsen zur Verfügung zu stellen, bei der die in der Vorrichtung von externen Störern verursachten Artefakte leicht heraus gefiltert werden können. Des Weiteren soll die Vorrichtungen in der Herstellung preiswert sein, so dass diese auch zur einmaligen Verwendung bei Patienten eingesetzt werden kann.

Diese Aufgabe wird gelöst mit einer Vorrichtung zur Erfassung und Übertragung von elektrischen Impulsen von der Körperoberfläche eines Patienten an eine die elektrischen Impulse verarbeitende Einrichtung, umfassend wenigstens zwei elektrische Leiter, von denen wenigstens zwei elektrische Leiter eine unterschiedliche Länge aufweisen, wenigstens eine die wenigstens zwei elektrischen Leiter umgebende elektrische Isolierung, einen Verbindungsanschluss zur Verbindung mit der die elektrischen Impulse verarbeitende Einrichtung vorzugsweise an einem Ende der wenigstens zwei elektrischen Leiter und eine Ausnehmung in der elektrischen Isolierung der wenigstens zwei elektrischen Leiter, so dass ein elektrischer Kontakt mit der Hautoberfläche des Patienten herstellbar ist, wobei bei wenigstens zwei Leitern mit unterschiedlicher Länge das Verhältnis aus dem elektrischen Widerstand und der Länge der Leiter und/oder das Verhältnis aus der Impedanz und der Länge der Leiter bei wenigstens einem kürzeren Leiter größer als bei wenigstens einem längeren Leiter ist.

Die von externen Störern induzierten Artefakte können damit von der die elektrischen Impulse verarbeitenden Einrichtung herausgefiltert werden, weil auch bei Leitern mit unterschiedlicher Länge ungefähr die gleichen Artefakte induziert werden.

Insbesondere weisen wenigstens zwei Leiter mit unterschiedlicher Länge im Wesentlichen den gleichen elektrischen Widerstand und/oder die gleiche Impedanz auf.

In einer zusätzlichen Ausführungsform unterscheiden sich bei wenigstens zwei Leitern mit unterschiedlicher Länge der elektrische Widerstand und/oder die Impedanz um weniger als 10 %, insbesondere um weniger als 1 bis 5 %.

In einer ergänzenden Ausgestaltung ist bei wenigstens zwei Leitern mit unterschiedlicher Länge bei wenigstens einem kürzeren Leiter die Querschnittsfläche kleiner als bei wenigstens einem längeren Leiter. Leiter mit einer größeren Länge weisen eine entsprechend skalierte größere Querschnittsfläche auf als Leiter mit einer kleineren Länge, so dass Leiter mit unterschiedlicher Länge beispielsweise im Wesentlichen über den gleichen elektrischen Widerstand verfügen.

Zweckmäßig ist bei wenigstens zwei Leitern mit unterschiedlicher Länge bei wenigstens einem kürzeren Leiter die Breite und/oder die Dicke kleiner als bei wenigstens einem längeren Leiter zu wählen. Leiter mit einer größeren Länge weisen eine entsprechend skalierte größere Breite und/oder Dicke auf als Leiter mit einer kleineren Länge, so dass Leiter mit unterschiedlicher Länge beispielsweise im Wesentlichen über den gleichen elektrischen Widerstand verfügen.

In einer weiteren Ausführungsform ist bei wenigstens zwei Leitern mit unterschiedlicher Länge bei wenigstens einem kürzeren Leiter der Querschnitt kleiner als bei wenigstens einem längeren Leiter. Leiter mit einer größeren Länge weisen einen entsprechend skalierten größeren Querschnitt auf als Leiter mit einer kleineren Länge, so dass Leiter mit unterschiedlicher Länge beispielsweise im Wesentlichen über den gleichen elektrischen Widerstand verfügen.

In einer ergänzenden Ausgestaltung bestehen die Leiter mit unterschiedlicher Länge aus unterschiedlichen Materialien mit einem entsprechend spezifischen Widerstand. Der spezifische Widerstand des Materials von kürzeren Leitern ist größer als der spezifische Widerstand des Materials von längeren Leitern. Dadurch kann beispielsweise der im Wesentlichen gleiche elektrische Widerstand bei Leitern mit unterschiedlicher Länge erreicht werden.

In einer zusätzlichen Ausgestaltung ist in wenigstens einem kürzeren Leiter ein elektrischer Widerstand, d. h. eine elektrisches oder elektronisches Bauteil, eingebaut. Dadurch kann beispielsweise der im Wesentlichen gleiche elektrische Widerstand bei Leitern mit unterschiedlicher Länge erreicht werden.
Vorzugsweise bestehen die Leiter aus einem Metall oder einer Legierung von Metallen, beispielsweise Aluminium, Kupfer, Gold, Silber oder Zinn.

In einer ergänzenden Ausgestaltung bestehen die Leiter aus leitfähigem Kunststoff, Graphit, Kohlenstofffasern oder metallisierten PA.

Vorzugsweise sind die Leiter aus einer Metallfolie oder einer Metallpaste gefertigt.

In einer weiteren Ausführungsform ist die Metallpaste gedruckt oder siebgedruckt.

In einer zusätzlichen Ausgestaltung sind die wenigstens zwei Leiter mit unterschiedlicher Länge mehrfach gedruckt oder siebgedruckt zur Erzielung einer unterschiedlichen Querschnittsfläche. Die unterschiedliche Querschnittsfläche der Leiter kann damit besonders einfach und kostengünstig bei der Herstellung der Leiter erreicht werden.

Vorzugsweise variiert der Abstand zwischen den Leitern, beispielsweise indem wenigstens ein Leiter in einem größeren Abstand zu anderen Leitern angeordnet ist als die anderen Leiter untereinander angeordnet sind oder ein Leiter gekrümmt, insbesondere als Schlangenlinie, ausgeführt ist. Insbesondere weist wenigstens ein Leiter einen wesentlich größeren Abstand auf als die anderen Leiter untereinander. Der wenigstens eine Leiter mit dem wesentlich größeren Abstand kann für andere Zwecke mit einem größeren Strombedarf genutzt werden als die anderen Leiter, die zum Leiten von schwachen Messsignalen dienen. Damit werden die von den Leitern für den größeren Strombedarf auf die anderen Leiter ausgehenden Störungen deutlich reduziert.

Zweckmäßig umgibt wenigstens ein Leiter wenigstens teilweise die anderen Leiter, so dass der wenigstens eine Leiter insbesondere bei einer Erdung zur Abschirmung von Störungen, insbesondere elektromagnetische Störungen, gegenüber den anderen Leitern dient.

In einer weiteren Ausführungsform sind zwei Leiter als Kondensatoren ausgebildet, insbesondere im Bereich des Verbindungsanschlusses, vorzugsweise dahingehend, dass die Oberfläche der Leiter entsprechend abgestimmt ist. Die Leiter werden beispielsweise breiter ausgeführt, um eine ausreichende Kapazität des Kondensators zu erhalten.

In einer ergänzenden Ausgestaltung ist wenigstens ein Leiter als Spule ausgeführt, vorzugsweise sind zwei Leiter mittels eines elektrischen Kontaktes zu einer elektrischen Spule verbunden, insbesondere im Bereich des Verbindungsanschlusses.

Zweckmäßig realisiert das Zusammenwirken des von den Leitern gebildeten Kondensators und der elektrischen Spule einen Filter, vorzugsweise einen Hoch-und/oder Tiefpassfilter, insbesondere einen Tiefpassfilter mit einer Grenzfrequenz unter 25 kHz. Damit können beispielsweise Artefakte aus HF-Chirurgiegeräten herausgefiltert werden.

In einer weiteren Ausgestaltung realisiert das Zusammenwirken des von den Leitern gebildeten Kondensators und der elektrischen Spule eine Antenne für einen RFID-Transponder.

Insbesondere ist der Verbindungsanschluss als Sender zur drahtlosen Übertragung von elektrischen Impulsen ausgeführt, insbesondere weist der Verbindungsanschluss einen RFID-Transponder auf.

In einer weiteren Ausführungsform ist an einer Ausnehmung in der elektrischen Isolierung der wenigstens zwei elektrischen Leiter der elektrische Kontakt mit der Hautoberfläche des Patienten mittels des wenigstens einen elektrischen Leiters unmittelbar herstellbar oder mittelbar herstellbar, indem eine Elektrode lösbar oder unlösbar an dem wenigstens einen elektrischen Leiter befestigbar ist.

In einer weiteren Ausführungsform besteht die Isolierung aus Kunststoff und/oder aus einem geschäumten und/oder aus einem textilen Material, z. B. aus Polyester, aus PE-Schaum oder aus einem synthetischen Webpelz. Polyester, vorzugsweise PE-Schaum, ermöglicht einen hohen Tragekomfort der Vorrichtung für den Patienten, da das Material durch eine Schäumung sehr weich ausgeführt werden kann. Weiterhin kann die Dielektrizitätskonstante der Vorrichtung durch Materialauswahl und Struktur des Materials angepasst werden. Die Vorrichtung kann so ausgeführt werden, dass eine geforderte Durchschlagfestigkeit von mindestens 5 kV eingehalten wird. Dies ist insbesondere bei einem gleichzeitigen Einsatz von Defibrilatoren an dem Patienten erforderlich.

In einer ergänzenden Ausführungsform umfasst die Vorrichtung ein mehrschichtiges flaches Band mit wenigstens zwei als Leiterbahnen ausgeführten Leitern und wenigstens eine erste und zweite nicht leitfähige Schicht als elektrische Isolierung für die Leiterbahnen, wobei die wenigstens zwei Leiterbahnen zwischen der ersten und zweiten nicht leitfähigen Schicht vorgesehen sind und vorzugsweise nebeneinander positioniert sind und wobei die erste und zweite nicht leitfähige Schicht derart miteinander verbunden sind, dass die wenigstens zwei Leiterbahnen isoliert zueinander angeordnet sind und vorzugsweise in dem Band und/oder in der wenigstens einen ersten und zweiten nicht leitfähigen Schicht wenigstens eine Ausnehmung im Bereich der Leiterbahnen vorgesehen ist, so dass ein Kontakt mit der Hautoberfläche des Patienten herstellbar ist.

Zweckmäßig ist eine dritte und/oder vierte leitfähige Schicht vorgesehen, wobei jeweils die dritte und/oder vierte leitfähige Schicht die erste und zweite nicht leitfähige Schicht umgibt. Damit werden in vorteilhafter Weise die von der Körperoberfläche eines Patienten abgeleiteten und über die Leiterbahnen der Vorrichtung übertragenen elektrischen Impulse gegen äußere Störungen, insbesondere elektromagnetische Störungen, abgeschirmt. Zur Erzielung einer optimalen Schirmung kann die dritte und vierte leitfähige Schicht in vorteilhafter Weise an wenigstens einer Position verbunden sein.

In einer weiteren Ausgestaltung bestehen die dritte und vierte leitfähige Schicht aus einem Netz leitender Fäden.

In einer zusätzlichen Ausführungsform weisen die leitenden Fäden zumindest in einem Abschnitt der jeweiligen dritten und vierten leitfähigen Schicht eine farbliche Markierung auf.

Insbesondere sind die Leiterbahnen an dem Verbindungsanschluss fixiert.

In einer weiteren Ausgestaltung ist wenigstens eine Perforation zwischen den isolierten Leiterbahnen vorgesehen. Die Leiterbahnen können dadurch voneinander getrennt und entsprechend den erforderlichen Ableitungspositionen auf der Körperoberfläche des Patienten positioniert werden. Die Perforation ist vorzugsweise als nacheinander angeordnete Langlöcher ausgeführt. Für diese Anwendung dienen die Langlöcher dazu, ein Auftrennen der Leiterbahnen ohne Beschädigung der Isolation zu ermöglichen. Damit wird eine Repositionierung der Elektroden möglich.

In einer ergänzenden Ausführungsform weist das Band eine Vielzahl von Falten auf, wobei die Falten mit vorzugsweise einem Winkel von 60° bis 120° zu den Leiterbahnen ausgerichtet sind. Dadurch wird eine Anordnung des Bandes in Form einer Ziehharmonika ermöglicht. Die Vorrichtung kann dem Anwender in einem gefalteten Zustand zur Verfügung gestellt werden, so dass der Anwender das Band entsprechend der Größe des Patienten auseinander ziehen kann.

Erfindungsgemäß wird die oben beschriebene Vorrichtung zur Übertragung von elektrischen Impulsen von der Körperoberfläche eines Patienten an einen Elektrokardiographen zur Erstellung eines Elektrokardiogramms, eine EIT Einrichtung oder eine Narkoseüberwachungseinrichtung des bispectralen Index verwendet.

Im Nachfolgenden wird ein Ausführungsbeispiel der Erfindung unter Bezugnahme auf die beigefügten Zeichnungen näher beschrieben. Es zeigt:
- Fig. 1: eine vereinfachte Darstellung der erfindungsgemäßen Vorrichtung in einer Draufsicht,
- Fig. 2: einen schematischen Querschnitt B-B gemäß Fig. 1 und
- Fig. 3: eine schematisierte Einzelheit A gemäß Fig. 1 in einem größeren Maßstab.

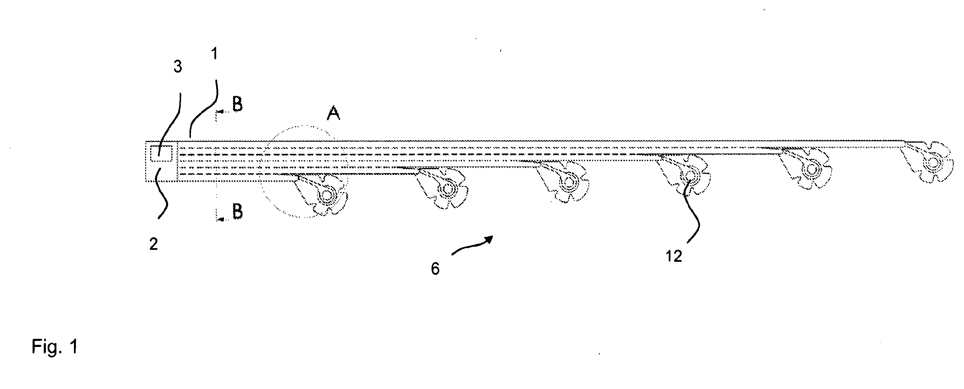

In Figur 1 ist die erfindungsgemäße Vorrichtung 6 zur Erfassung und Übertragung von elektrischen Impulsen von der Körperoberfläche eines Patienten beispielsweise an einen Elektrokardiographen als eine elektrische Impulse verarbeitende Einrichtung (nicht dargestellt) gezeigt. Die Vorrichtung 6 umfasst ein mehrschichtiges Band 1 mit einem Verbindungsanschluss 2. Der Verbindungsanschluss 2 ist vorgesehen, um die elektrischen Impulse direkt oder über einen Adapter an den Elektrokardiographen zu übertragen. In einer weiteren Ausführungsform kann der Verbindungsanschluss 2 als Sender zur drahtlosen Übertragung der elektrischen Impulse ausgeführt sein. Vorzugsweise weist der Verbindungsanschluss 2 einen RFID-Transponder 3 auf, in dem Kennwerte der Vorrichtung 6 hinterlegt sind, so dass eine automatische Identifizierung der Vorrichtung 6 an dem Elektrokardiographen erfolgen kann. Es können aber auch Kennwerte der Patienten in dem RFID-Transponder 3 hinterlegt werden, die eine eindeutige Zuordnung der elektrischen Impulse an beispielsweise vorhergehend in dem Elektrokardiographen hinterlegte Daten gestattet.

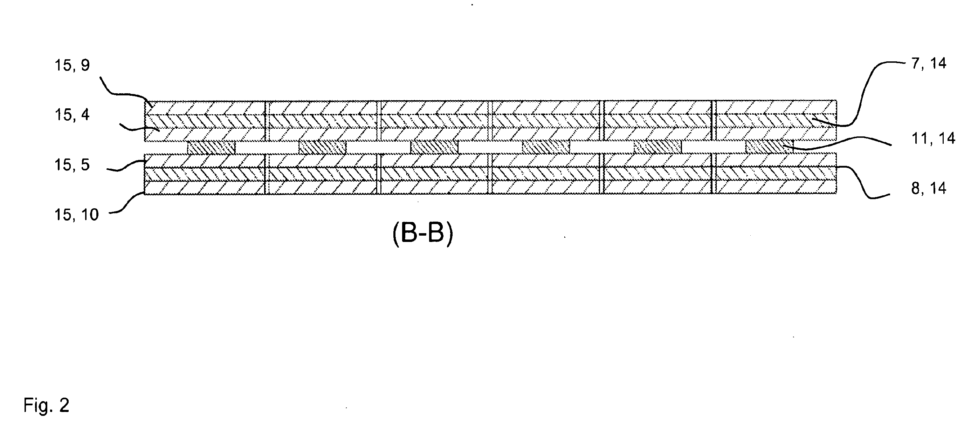

Das Band 1 ist mit einer ersten und zweiten nicht leitfähigen Schicht 4, 5 (Fig. 2) aus Polyester, vorzugsweise PE-Schaum, ausgeführt. Die erste und zweite nicht leitfähige Schicht 4, 5 kann auch aus Polypropylen oder Polyurethan bestehen. Zwischen der ersten und zweiten nicht leitfähigen Schicht 4, 5 sind fünf als Leiterbahnen 11 ausgeführte Leiter 14 nebeneinander angeordnet. Die ersten und zweiten nicht leitfähigen Schicht 4, 5 ist als Isolierung 15 vorgesehen. Die erste und zweite nicht leitfähige Schicht 4, 5 sind derart miteinander verbunden, dass die fünf Leiterbahnen 11 isoliert zueinander angeordnet sind. Die Leiterbahnen 11 sind an dem Verbindungsanschluss 2 fixiert (Fig. 2). In dem Band 1 ist jeweils eine Ausnehmung in dem Band 1 und/oder der nicht leitfähigen Schicht 4, 5 vorgesehen, so dass ein Kontakt jeder Leiterbahn 11 mit der Haut des Patienten hergestellt werden kann. Die Leiterbahnen 11 sind jeweils in dem Bereich der Ausnehmung großflächig in Form einer Elektrode 12 ausgeführt. Die Leiterbahnen 11 können in einer weiteren Ausführungsform eine Verbindungsmöglichkeit mit jeweils einer separaten Elektrode 12 aufweisen (nicht dargestellt).

Die Leiterbahnen 11 weisen eine unterschiedliche Länge auf, weil diese, siehe Fig. 1, die Elektroden 12 in einem unterschiedlichen Abstand mit dem Verbindungsanschluss 2 verbinden. Die Leiterbahnen 11 bestehen aus einer im Siebdruckverfahren auf die erste oder zweite nicht leitfähige Schicht 4, 5 aufgedruckte Silberleitpaste. Durch mehrfaches Bedrucken mit der gleichen Schablone kann die Dicke der Leiterbahnen 11 variiert werden. Die Dicke der Leiterbahnen 11 ist dahingehend ausgelegt (nicht dargestellt), dass der elektrische Widerstand und/oder die elektrische Impedanz der Leiterbahnen 11 zwischen dem Verbindungsanschluss 2 und den Elektroden 12 im Wesentlichen gleich ist.

In einer weiteren Ausführungsform ist die Querschnittsfläche, beispielsweise mittels einer variablen Breite, der Leiterbahnen 11 dahingehend ausgelegt (nicht dargestellt), dass der elektrische Widerstand und/oder die elektrische Impedanz der Leiterbahnen 11 zwischen dem Verbindungsanschluss 2 und den Elektroden 12 im Wesentlichen gleich ist. In einer ergänzenden Ausführungsform werden die Leiterbahnen 11 mit einer entsprechend skalierten Querschnittsfläche auf die erste nicht leitfähige Schicht 4 aus Kunststofffasern aufgelegt, anschließend die zweite nicht leitfähige Schicht 5 aufgelegt und darauf folgend die erste und zweite nicht leitfähige Schicht 4, 5 durch Heißlaminieren verbunden.

In den Leiterbahnen 11 können durch externe Störer, beispielsweise einem HF-Chirurgiegerät oder einem Mobiltelefon, elektrische Ströme, im folgenden Artefakte genannt, induziert werden. Diese Artefakte sind in den Leiterbahnen 11 mit unterschiedlicher Länge aufgrund des im Wesentlichen gleichen elektrischen Widerstandes und/oder der im Wesentlichen gleichen elektrischen Impedanz der Leiterbahnen 11 cirka gleich groß. Dies ermöglicht problemlos eine vollständige oder nahezu vollständige Filterung der Artefakte mittels beispielsweise in dem Elektrokardiographen hinterlegter Algorithmen. Die medizinische Auswertung und Analyse der Messsignale, beispielsweise einer EKG-Messung, kann damit sicher und zuverlässig auch bei externen Störern vorgenommen werden, weil der Elektrokardiograph die induzierten Artefakte herausfiltert kann.

Die Vorrichtung 6 kann aufgrund ihres konstruktiven Aufbaus einfach und preiswert hergestellt und in Krankenhäusern zur einmaligen Verwendung bei Patienten eingesetzt werden. Damit können auch bei derartigen Vorrichtungen 6 EKG-, EEG- und EIT-Messungen ohne die verzerrenden Einflüsse von Störern durchgeführt werden.

In einer weiteren, nicht dargestellten Ausführungsform können die Leiterbahnen 11 an dem Verbindungsanschluss 2 als Spule ausgeführt sein oder eine Spule mit wenigstens einer Leiterbahn 11 verbunden sein. Die elektrische Verbindung zwischen zwei mittels des Siebdruckverfahrens hergestellten Leiterbahnen 11 zu einer Spule wird mit einem Durchkontaktierungsloch, einem so genannten Vias, ausgeführt. Im Siebdruckverfahren wird die Silberleitpaste auch in das Vias eingebracht, so dass eine elektrische Verbindung zwischen wenigstens zwei Leiterbahnen 11 besteht. Zudem können wenigstens zwei Leiterbahnen 11 als Kondensator ausgeführt sein. Die Kapazität der Kondensatoren kann durch eine entsprechend ausgelegte Breite und/oder Länge der Leiterbahnen 11 angepasst werden. Der Kondensator kann mit einer Spule verbunden sein und einen elektrischen Schwingkreis bilden. Insbesondere dient der elektrische Schwingkreis als Filter zur selektiven Filterung von elektrischen Impulsen, beispielsweise als Tiefpassfilter für Frequenzen unter 25 kHz.

In einer nicht dargestellten Ausführungsform kann in der ersten und/oder zweiten nicht leitfähigen Schicht 4, 5 ein Barcode, vorzugsweise im Bereich des Verbindungsanschlusses 2, integriert sein. Dies ermöglicht eine eindeutige Identifikation der Vorrichtung 6 und erleichtert insbesondere die Logistik.

In Fig. 2 ist der Aufbau des mehrschichtigen flachen Bandes 1 gezeigt. Um die erste und zweite nicht leitfähige Schicht 4 und 5 ist eine dritte und vierte leitfähige Schicht 7 und 8 vorgesehen, die vorzugsweise aus einem Netz leitender Fäden bestehen. Die leitenden Fäden können mit einer farblichen Markierung ausgeführt sein und damit eine Zuordnung der einzelnen Elektroden 12 der jeweiligen Leiterbahnen 11 zu den Ableitungspositionen der elektrischen Impulse von der Körperoberfläche des Patienten erleichtern. Mit der dritten und vierten leitfähigen Schicht 7 und 8 werden in vorteilhafter Weise die von der Körperoberfläche des Patienten abgeleiteten und über die Leiterbahnen 11 der Vorrichtung 6 übertragenen elektrischen Impulse gegen äußere Störungen, insbesondere elektromagnetische Störungen, abgeschirmt. Die dritte und vierte leitfähige Schicht 7 und 8 kann dabei in vorteilhafter Weise an wenigstens einer Position miteinander verbunden werden, um eine Optimierung der Schirmung zu erreichen und ist im Allgemeinen geerdet.

Die Außenflächen des Bandes 1 werden, wie in Fig. 2 dargestellt, von einer fünften und sechsten nicht leitfähigen Schicht 9 und 10 als weitere Isolierung 15 gebildet. An der äußeren fünften und sechsten nicht leitfähigen Schicht 9 und 10 des Bandes 1 sind im Bereich der Elektroden 12 Haftelemente zum Haften der Elektroden 12 an der Hautoberfläche vorgesehen (nicht dargestellt). Die nicht leitfähigen Schichten 4, 5, 9 und 10 sind vorzugsweise aufgrund ihrer Dicke dahingehend ausgelegt, dass diese einen Überspannungsschutz im Bereich von fünf Kilovolt gewährleisten.

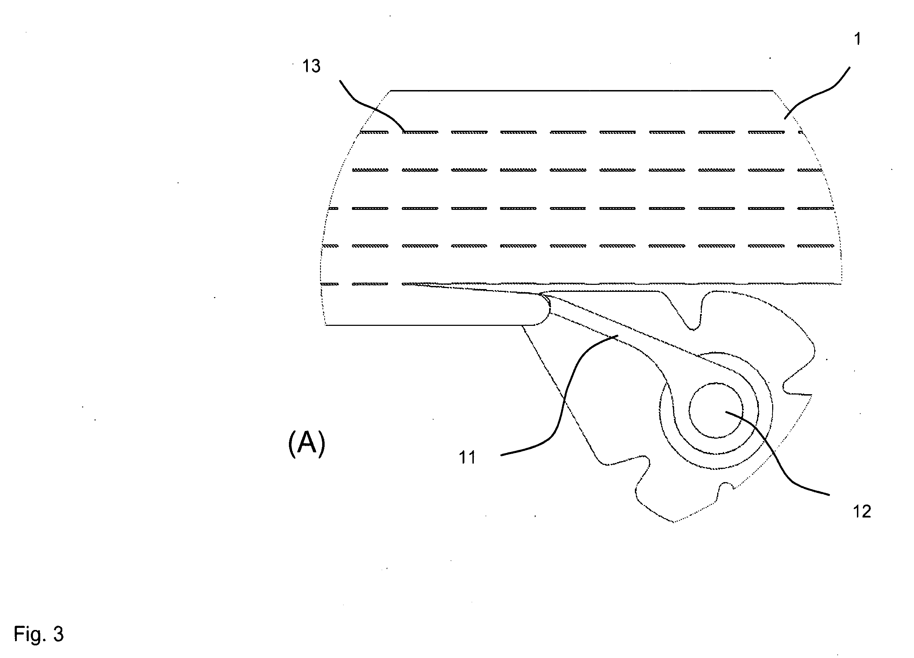

In Fig. 3 sind Perforationen 13 zwischen den isolierten Leiterbahnen 11 dargestellt, die dazu bestimmt sind, die Leiterbahnen 11 partiell voneinander zu trennen und entsprechend den erforderlichen Ableitungspositionen auf der Körperoberfläche des Patienten positionieren zu können. Vorzugsweise sind die Perforationen 13 als Langlöcher zwischen den isolierten Leiterbahnen 11 ausgeführt. Damit wird ein Auftrennen der Leiterbahnen 11 ohne Beschädigung der Isolation und somit eine Repositionierung der Elektroden 12 ermöglicht. In einer nicht dargestellten Ausführung weist das Band 1 eine Vielzahl von rechtwinklig zu den Leiterbahnen 11 vorgesehenen Falten auf. Diese Falten ermöglichen eine Anordnung des Bandes 1 in Form einer Ziehharmonika. Die Vorrichtung 6 kann dem Anwender in einem gefalteten Zustand zur Verfügung gestellt werden. Der Anwender kann die Vorrichtung 6 entsprechend der Größe des Patienten auseinander ziehen, und somit die Elektroden 12 an genau definierte anatomische Positionen an der Körperoberfläche des Patienten anbringen.

Insgesamt betrachtet stellt die in den Figuren 1 bis 3 dargestellte Ausführungsvariante der erfindungsgemäße Vorrichtung 6 ein EKG-, EEG- und EIT-Kabel mit integrierten Elektroden 12 zur Verfügung, das klinische Prozesse vereinfacht, da es wegen optimierter Herstellungskosten pro Patient nur einmal verwendet wird, den Patienten durch die Care-Areas von der Notfallbehandlung über die OP bis in den Intensivbereich begleiten kann und zudem die von Störern induzierten Artefakte im Elektrokardiographen problemlos heraus gefiltert werden können, so dass eine medizinisch sichere und zuverlässige Analyse der Messsignale möglich ist.

### BEZUGSZEICHENLISTE

- 1: Band
- 2: Verbindungsanschluss
- 3: RFID-Transponder
- 4: Erste nicht leitfähige Schicht
- 5: Zweite nicht leitfähige Schicht
- 6: Vorrichtung
- 7: Dritte leitfähige Schicht
- 8: Vierte leitfähige Schicht
- 9: Fünfte nicht leitfähige Schicht
- 10: Sechste nicht leitfähige Schicht
- 11: Leiterbahn
- 12: Elektrode
- 13: Perforation
- 14: Leiter
- 15: Isolierung

## Patentansprüche

1. Vorrichtung zur Erfassung und Übertragung von elektrischen Impulsen von der Körperoberfläche eines Patienten an eine die elektrischen Impulse verarbeitende Einrichtung, umfassend
- wenigstens zwei elektrische Leiter (7, 8, 11, 14), von denen wenigstens zwei elektrische Leiter (11, 14) eine unterschiedliche Länge aufweisen, wobei bei den wenigstens zwei Leitern (11, 14) mit unterschiedlicher Länge das Verhältnis aus dem elektrischen Widerstand und der Länge der Leiter (11, 14) und/oder das Verhältnis aus der Impedanz und der Länge der Leiter (11, 14) bei wenigstens einem kürzeren Leiter (11, 14) größer ist als bei wenigstens einem längeren Leiter (11, 14),
- einen Verbindungsanschluss (2) zur Verbindung mit der die elektrischen Impulse verarbeitende Einrichtung,
- wenigstens eine die wenigstens zwei elektrischen Leiter (7, 8, 11, 14) umgebende elektrische Isolierung (4, 5, 9, 10, 15),
- eine Ausnehmung in der elektrischen Isolierung (4, 5, 9, 10, 15) der weinigstens zwei elektrischen Leiter (11, 14), so dass ein elektrischer Kontakt mit der Hautoberfläche des Patienten herstellbar ist,

2. Vorrichtung nach Anspruch 1, **dadurch gekennzeichnet, dass** wenigstens zwei Leiter (11, 14) mit unterschiedlicher Länge im Wesentlichen den gleichen elektrischen Widerstand und/oder im Wesentlichen die gleiche Impedanz aufweisen.

3. Vorrichtung nach Anspruch 1 oder 2, **dadurch gekennzeichnet, dass** sich bei wenigstens zwei Leitern (11, 14) mit unterschiedlicher Länge der elektrische Widerstand und/oder die Impedanz um weniger als 10 %, insbesondere um weniger als 1 bis 5 %, unterscheidet.

4. Vorrichtung nach einem oder mehreren der vorhergehenden Ansprüche, **dadurch gekennzeichnet, dass** bei wenigstens zwei Leitern (11, 14) mit unterschiedlicher Länge bei wenigstens einem kürzeren Leiter (11, 14) die Querschnittsfläche kleiner ist als bei wenigstens einem längeren Leiter (11, 14).

5. Vorrichtung nach einem oder mehreren der vorhergehenden Ansprüche, **dadurch gekennzeichnet, dass** bei wenigstens zwei Leitern (11, 14) mit unterschiedlicher Länge bei wenigstens einem kürzeren Leiter (11, 14) die Breite und/oder die Dicke kleiner ist als bei wenigstens einem längeren Leiter (11,14).

6. Vorrichtung nach einem oder mehreren der vorhergehenden Ansprüche, **dadurch gekennzeichnet, dass** bei wenigstens zwei Leitern (11, 14) mit unterschiedlicher Länge bei wenigstens einem kürzeren Leiter (11, 14) der Querschnitt kleiner ist als bei wenigstens einem längeren Leiter (11, 14).

7. Vorrichtung nach einem oder mehreren der vorhergehenden Ansprüche, **dadurch gekennzeichnet, dass** die wenigstens zwei Leiter (7, 8, 11, 14) aus einem Metall oder einer Legierung von Metallen, beispielsweise Aluminium, Kupfer, Gold, Silber oder Zinn, bestehen.

8. Vorrichtung nach Anspruch 7, **dadurch gekennzeichnet, dass** die wenigstens zwei Leiter (7, 8, 11, 14) aus einer Metallfolie oder einer Metallpaste gefertigt sind, wobei die Metallpaste vorzugsweise gedruckt oder siebgedruckt ist.

9. Vorrichtung nach einem oder mehreren der Ansprüche 1 bis 6, **dadurch gekennzeichnet, dass** die wenigstens zwei Leiter (7, 8, 11, 14) aus leitfähigem Kunststoff, Graphit, Kohlenstofffasern oder metallisierten PA bestehen.

10. Vorrichtung nach einem oder mehreren der vorhergehenden Ansprüche, **dadurch gekennzeichnet, dass** der Abstand zwischen den wenigstens zwei Leitern (7, 8, 11, 14) variiert, beispielsweise indem wenigstens ein Leiter (7, 8, 11, 14) in einem größeren Abstand zu anderen Leitern (7, 8, 11, 14) angeordnet ist als die anderen Leiter (7, 8, 11, 14) untereinander angeordnet sind oder ein Leiter (7, 8, 11, 14) gekrümmt, insbesondere als Schlangenlinie, ausgeführt ist.

11. Vorrichtung nach einem oder mehreren der vorhergehenden Ansprüche, **dadurch gekennzeichnet, dass** wenigstens zwei Leiter (7, 8, 11, 14) als Kondensatoren ausgebildet sind, insbesondere im Bereich des Verbindungsanschlusses (2), vorzugsweise dahingehend, dass die Oberfläche der Leiter (7, 8, 11, 14) entsprechend abgestimmt ist.

12. Vorrichtung nach einem oder mehreren der vorhergehenden Ansprüche, **dadurch gekennzeichnet, dass** ein Leiter (7, 8, 11, 14) als Spule ausgeführt ist, vorzugsweise zwei Leiter (7, 8, 11, 14) mittels eines elektrischen Kontaktes zu einer elektrischen Spule verbunden sind, insbesondere im Bereich des Verbindungsanschlusses (2).

13. Vorrichtung nach Anspruch 11 und 12, **dadurch gekennzeichnet, dass** das Zusammenwirken des von zwei Leitern (7, 8, 11, 14) gebildeten Kondensators und der elektrischen Spule einen Filter, insbesondere einen Tiefpassfilter mit einer Grenzfrequenz unter 25 kHz, realisiert.

14. Vorrichtung nach Anspruch 11 und 12 und/oder 13, **dadurch gekennzeichnet, dass** das Zusammenwirken des von den Leitern (7, 8, 11, 14) gebildeten Kondensators und der elektrischen Spule eine Antenne für einen RFID-Transponder realisiert.

15. Vorrichtung nach einem oder mehreren der vorhergehenden Ansprüche, **dadurch gekennzeichnet, dass** der Verbindungsanschluss (2) als Sender zur drahtlosen Übertragung von elektrischen Impulsen ausgeführt ist, insbesondere der Verbindungsanschluss einen RFID-Transponder (3) aufweist.

16. Vorrichtung nach einem oder mehreren der vorhergehenden Ansprüche, **dadurch gekennzeichnet, dass** an einer Ausnehmung in der elektrischen Isolierung (4, 5, 9, 10, 15) der wenigstens zwei elektrischen Leiter (11, 14) der elektrische Kontakt mit der Hautoberfläche des Patienten mittels des wenigstens einen elektrischen Leiters (11, 14) unmittelbar herstellbar ist oder mittelbar herstellbar ist, indem eine Elektrode (12) lösbar oder unlösbar an dem wenigstens einen elektrischen Leiter (11, 14) befestigbar ist.

17. Vorrichtung nach einem oder mehreren der vorhergehenden Ansprüche, **dadurch gekennzeichnet, dass** die Isolierung (4, 5, 9, 10, 15) aus Kunststoff und/oder aus einem geschäumten und/oder aus textilen Material, z. B. aus Polyester, aus PE-Schaum oder aus einem synthetischen Webpelz, besteht.

## Claims

1. Device for detecting and transmitting electrical pulses from the surface of a patient's body to a means which processes the electrical pulses, comprising
- at least two electrical conductors (7, 8, 11, 14), of which at least two electrical conductors (11, 14) are of different lengths, the ratio of the resistance of the conductors (11, 14) to their length and/or the ratio of the impedance of the conductors (11, 14) to their length being, in the case of these at least two electrical conductors (11, 14) of different lengths, greater in the case of at least a shorter conductor (11, 14) than in the case of at least a longer conductor (11, 14),
- a connecting port (2) for connection to the means which processes the electrical pulses,
- at least one electrical insulating material (4, 5, 9, 10, 15) surrounding the at least two electrical conductors (7, 8, 11, 14),
- an opening in the electrical insulating material (4, 5, 9, 10, 15) insulating the at least two electrical conductors (11, 14), thus enabling electrical contact to be made with the surface of the patient's skin.

2. Device according to claim 1, **characterised in that** at least two conductors (11, 14) of different lengths have substantially the same resistance and/or substantially the same impedance.

3. Device according to claim 1 or 2, **characterised in that**, in the case of at least two conductors (11, 14) of different lengths, the resistance and/or the impedance differs by less than 10% and in particular by less than 1 to 5%.

4. Device according to one or more of the preceding claims, **characterised in that**, in the case of at least two conductors (11, 14) of different lengths, the cross-sectional area is larger in the case of at least a shorter conductor (11, 14) than in the case of at least a longer conductor (11, 14).

5. Device according to one or more of the preceding claims, **characterised in that**, in the case of at least two conductors (11, 14) of different lengths, the width and/or thickness is smaller in the case of at least a shorter conductor (11, 14) than in the case of at least a longer conductor (11, 14).

6. Device according to one or more of the preceding claims, **characterised in that**, in the case of at least two conductors (11, 14) of different lengths, the cross-section is smaller in the case of at least a shorter conductor (11, 14) than in the case of at least a longer conductor (11, 14).

7. Device according to one or more of the preceding claims, **characterised in that** the at least two conductors (7, 8, 11, 14) are composed of a metal or an alloy of metals, such for example as aluminium, copper, gold, silver or tin.

8. Device according to claim 7, **characterised in that** the at least two conductors are produced from a metal foil or a metal paste, the metal paste preferably being printed or screen printed.

9. Device according to one or more of claims 1 to 6, **characterised in that** the at least two conductors (7, 8, 11, 14) are composed of conductive plastics material, graphite, carbon fibres or metallised polyamide (PA).

10. Device according to one or more of the preceding claims, **characterised in that** the distance between the at least two conductors varies, such for example as in that at least one conductor (7, 8, 11, 14) is arranged at a greater distance from the other conductors (7, 8, 11, 14) than the other conductors (7, 8, 11, 14) are arranged from one another, or one conductor (7, 8, 11, 14) is produced to be curved and in particular to follow a serpentine line.

11. Device according to one or more of the preceding claims, **characterised in that** at least two conductors (7, 8, 11, 14) take the form of capacitors, particularly in the region of the connecting port (2), preferably to the effect that the surfaces of the conductors (7, 8, 11, 14) are suitably tuned.

12. Device according to one or more of the preceding claims, **characterised in that** one conductor (7, 8, 11, 14) takes the form of a coil and two conductors are preferably connected by means of an electrical contact to form an electrical coil, particularly in the region of the connecting port (2).

13. Device according to claims 11 and 12, **characterised in that** the co-operation of the capacitor formed by two conductors (7, 8, 11, 14) and of the electrical coil produces a filter, and in particular a low-pass filter having a cut-off frequency of less than 25 kHz.

14. Device according to claims 11 and 12 and/or 13, **characterised in that** the co-operation of the capacitor formed by the conductors (7, 8, 11,14) and of the electrical coil produces an aerial for an RFID transponder.

15. Device according to one or more of the preceding claims, **characterised in that** the connecting port (2) takes the form of a transmitter for the wireless transmission of electrical pulses, and in particular the connecting port has an RFID transponder (3).

16. Device according to one or more of the preceding claims, **characterised in that** the electrical contact with the surface of the patient's skin can be made directly or can be made indirectly by means of the at least one electrical conductor (11, 14), at an opening in the electrical insulating material (4, 5, 9, 10, 15) insulating the at least two electrical conductors (11, 14), by virtue of the fact that an electrode (12) can be detachably or non-detachably fastened to the at least one electrical conductor (11, 14).

17. Device according to one or more of the preceding claims, **characterised in that** the insulating material (4, 5, 9, 10, 15) is composed of plastics material and/or of a foamed material and/or of a textile material, e.g. polyester, of polyethylene (PE) foam or of a synthetic woven fur.

## Revendications

1. Dispositif pour détecter et transmettre des impulsions électriques de la surface du corps d'un patient à un dispositif traitant les impulsions électriques, comprenant
- au moins deux conducteurs (7, 8, 11, 14) électriques, dont au moins deux conducteurs (11, 14) électriques présentent une longueur différente, sachant que, pour les au moins deux conducteurs (11, 14) présentant une longueur différente, le rapport de la résistance électrique et de la longueur des conducteurs (11, 14) et/ou le rapport de l'impédance et de la longueur des conducteurs (11, 14) dans le cas d'au moins un conducteur (11, 14) plus court est plus grand que dans le cas d'au moins un conducteur (11, 14) plus long,
- un branchement de liaison (2) pour la liaison avec le dispositif traitant les impulsions électriques,
- au moins une isolation (4, 5, 9, 10, 15) électrique entourant les au moins deux conducteurs (7, 8, 11, 14) électriques,
- un évidement dans l'isolation (4, 5, 9, 10, 15) électrique des au moins deux conducteurs (11, 14) électriques, de sorte qu'un contact électrique peut être établi avec la surface de la peau du patient.

2. Dispositif selon la revendication 1, **caractérisé en ce qu'**au moins deux conducteurs (11, 14) présentant une longueur différente présentent sensiblement la même résistance électrique et/ou sensiblement la même impédance.

3. Dispositif selon la revendication 1 ou 2, **caractérisé en ce que**, dans le cas d'au moins deux conducteurs (11, 14) présentant une longueur différente, la résistance électrique et/ou l'impédance diffère(nt) de moins de 10 %, en particulier de moins de 1 à 5 %.

4. Dispositif selon l'une quelconque ou plusieurs des revendications précédentes, **caractérisé en ce que**, dans le cas d'au moins deux conducteurs (11, 14) présentant une longueur différente avec au moins un conducteur (11, 14) plus court, la surface de section est plus petite que dans le cas d'au moins un conducteur (11, 14) plus long.

5. Dispositif selon l'une quelconque ou plusieurs des revendications précédentes, **caractérisé en ce que**, dans le cas d'au moins deux conducteurs (11, 14) présentant une longueur différente avec au moins un conducteur (11, 14) plus court, la largeur et/ou l'épaisseur est/sont plus petite(s) que dans le cas d'au moins un conducteur (11, 14) plus long.

6. Dispositif selon l'une quelconque ou plusieurs des revendications précédentes, **caractérisé en ce que**, dans le cas d'au moins deux conducteurs (11, 14) présentant une longueur différente avec au moins un conducteur (11, 14) plus court, la section est plus petite que dans le cas d'au moins un conducteur (11, 14) plus long.

7. Dispositif selon l'une quelconque ou plusieurs des revendications précédentes, **caractérisé en ce que** les au moins deux conducteurs (7, 8, 11, 14) sont à base d'un métal ou d'un alliage de métaux, par exemple de l'aluminium, du cuivre, de l'argent ou de l'étain.

8. Dispositif selon la revendication 7, **caractérisé en ce que** les au moins deux conducteurs (7, 8, 11, 14) sont fabriqués à base d'une feuille de métal ou d'une pâte de métal, la pâte de métal étant de préférence imprimée ou sérigraphiée.

9. Dispositif selon l'une quelconque ou plusieurs des revendications 1 à 6, **caractérisé en ce que** les au moins deux conducteurs (7, 8, 11, 14) sont à base de matière synthétique conductible, graphite, fibres de carbone ou de polyamides métallisés.

10. Dispositif selon l'une quelconque ou plusieurs des revendications précédentes, **caractérisé en ce que** la distance entre les au moins deux conducteurs (7, 8, 11, 14) varie, par exemple du fait qu'au moins un conducteur (7, 8, 11, 14) est disposé à une distance plus grande des autres conducteurs (7, 8, 11, 14) que les autres conducteurs (7, 8, 11, 14) entre eux ou un conducteur (7, 8, 11, 14) est réalisé de façon incurvée, en particulier sous forme de ligne sinueuse.

11. Dispositif selon l'une quelconque ou plusieurs des revendications précédentes, **caractérisé en ce qu'**au moins deux conducteurs (7, 8, 11, 14) sont conçus sous forme de condensateurs, en particulier dans la zone du branchement de liaison (2), de préférence en ce sens que la surface des conducteurs (7, 8, 11, 14) est adaptée en conséquence.

12. Dispositif selon l'une quelconque ou plusieurs des revendications précédentes, **caractérisé en ce qu'**un conducteur (7, 8, 11, 14) est conçu sous forme de bobine, de préférence deux conducteurs (7, 8, 11, 14) sont reliés au moyen d'un contact électrique pour former une bobine électrique, en particulier dans la zone du branchement de liaison (2).

13. Dispositif selon les revendications 11 et 12, **caractérisé en ce que** l'action conjuguée du condensateur formé par deux conducteurs (7, 8, 11, 14) et de la bobine électrique réalise un filtre, en particulier un filtre passe-bas avec une fréquence limite inférieure à 25 kHz.

14. Dispositif selon les revendications 11 et 12 et/ou 13, **caractérisé en ce que** l'action conjuguée du condensateur formé par les conducteurs (7, 8, 11, 14) et de la bobine électrique réalise une antenne pour un transpondeur RFID.

15. Dispositif selon l'une quelconque ou plusieurs des revendications précédentes, **caractérisé en ce que** le branchement de liaison (2) est réalisé sous forme d'émetteur pour la transmission sans fil d'impulsions électriques, en particulier le branchement de liaison présente un transpondeur RFID (3).

16. Dispositif selon l'une quelconque ou plusieurs des revendications précédentes, **caractérisé en ce que**, sur un évidement dans l'isolation (4, 5, 9, 10, 15) électrique des au moins deux conducteurs (11, 14) électriques, le contact électrique avec la surface de la peau du patient peut être établi directement ou indirectement au moyen du au moins un conducteur (11, 14) électrique en ce sens qu'une électrode (12) peut être fixée de façon amovible ou inamovible sur le au moins un conducteur (11, 14) électrique.

17. Dispositif selon l'une quelconque ou plusieurs des revendications précédentes, **caractérisé en ce que** l'isolation (4, 5, 9, 10, 15) à base de matière synthétique et/ou d'un matériau moussé et/ou de matériau textile, par exemple du polyester, de mousse de polyéthylène ou d'une fourrure tissée synthétique.
